Europäisches Patentamt

European Patent Office

Office européen des brevets

(11) Publication number : **0 485 210 A2**

# (12) EUROPEAN PATENT APPLICATION

(21) Application number : **91310298.4**

(51) Int. Cl.<sup>5</sup> : **A61L 27/00, A61L 15/32**

(22) Date of filing : **07.11.91**

(30) Priority : **08.11.90 US 610542**

(43) Date of publication of application :
**13.05.92 Bulletin 92/20**

(84) Designated Contracting States :
**AT BE CH DE DK ES FR GB GR IT LI LU NL SE**

(71) Applicant : **MATRIX PHARMACEUTICAL INC.**
**1430 O'Brien Drive Suite H**
**Menlo Park California 94025 (US)**

(72) Inventor : **Sierra, David H.**
**723 Menlo Avenue, 2**
**Menlo Park, California 94025 (US)**
Inventor : **Luck, Edward E.**
**216 Robin Way**
**Menlo Park, California 94025 (US)**
Inventor : **Brown, Dennis M.**
**100 San Mateo Drive**
**Menlo Park, California 94025 (US)**

(74) Representative : **Hardisty, David Robert et al**
**BOULT, WADE & TENNANT 27 Furnival Street**
**London EC4A IPQ (GB)**

(54) **Fibrin/collagen membrane material for biomedical use.**

(57)  Gel membranes are provided by gelling fibrinogen with thrombin by itself or in combination with gelation of collagen. After dehydration, the resulting membranes may be used as dressings, temporary skin grafts, or the like. The gels may be used without dehydration for drug delivery or tissue regeneration or growth inhibition.

EP 0 485 210 A2

Technical Field

The field of this invention concerns proteinaceous membranes for use in application to epithelial tissue, drug release, tissue separation or wrapper, or the like.

Background

The skin is a major factor in protecting the body against disease. The healing of wounds, skin grafts, and prior structural breakdown of epithelial tissue is a slow process, during which protection is compromised. Burns, incisions, and abrasions reduce the effectiveness of the skin in preventing invasion of various pathogens, thus exposing the patient to disease. Numerous dressings and drugs have been developed and continue to be developed to substitute for the natural skin protection, while such protection is compromised. There is, therefore, substantial interest in developing products which will enhance the rate of healing of a wound or other epithelial damage, while affording protection against pathogenic invasion.

Implantable drug delivery devices utilizing biodegradable, bicompatible polymeric materials are of paramount interest in providing sustained release of pharmacologic agents both locally and systemically. Biodegradability obviates the need to surgically remove the implant once the dose has been released. Biocompatibility encompasses not only toxicity issues but inflammation processes and immunologic responses as well. These may interfere with the drug release, decrease dosing efficacy and therefore adequate disease contact.

Relevant Literature

U.S. Patent No. 4,418,691 describes a fibrous lattice of viable cells, which may be grafted onto a wound bed. Other references of interest are Harvey, Ann. Surg. (1918) 68:66; Ferry and Morrison, J. Clin. Invest., (1944) 23:566; Dillon et al., Biotechnol. Bioengr., (1976), 18:133; Morrison and Linder, J. Clin. Invest. (1944), 23:573; Griffith et al., Trans Soc. Biomat., (1988), 11:58; Aprahamian et al., J. Biomed. Mat. Res., (1987) 2:1965; Linder et al., U.S. Patent No. 4,600,574; Osbahr, Biomaterials (1980) 1:100; Kovacs et al., Proc. 1st World Biomat. Congress, Eur. Soc. Biomat. Vienna, 1980.

SUMMARY OF THE INVENTION

Fibrous membranes are provided comprising at least a portion of fibrin and optionally collagen, where the proteins are polymerized into a gel, optionally compressed and dried. The compressed gel may be optionally dried in a stretched configuration. Upon rewetting, the membrane returns to its original conformation. Increasing proportions of fibrin, as well as decreased amounts of thrombin, provide for increased elasticity. Alternatively, the material may be left in a gel form.

DESCRIPTION OF THE SPECIPIC EMBODIMENT

Methods and compositions are provided for producing proteinaceous membranes. The membranes are comprised at least in part of fibrin and may include a major portion of collagen. In addition, various therapeutic agents may be combined in the membrane depending upon the contemplated use of the membrane.

In preparing the membrane, various techniques may be employed for causing the polymerization. A fibrin membrane may be readily obtained by adding a small amount of thrombin to fibrinogen in a buffered salt solution. Generally, for 100 mg of fibrinogen, from about 1 to 100, preferably from about 5 to 20 NIH units of thrombin are employed. The salt solution will generally be from about 10 to 100 weight % of the fibrinogen present in the solution, preferably about 20 to 50 weight % of the fibrinogen. The thrombin may be added at a reduced temperature, generally not less than about 2°C and up to about 37°C, more usually about 5°C.

If desired, up to about 100 weight %, preferably not more than about 50 weight %, of total protein may be collagen, particularly an acidic collagen solution (ACS) or insoluble collagen fibrils generally being in a weight ratio of fibrinogen to collagen of 1:0-3, usually 1:0.5-1. The collagen may be from any mammalian source, e.g., bovine, porcine, ovine, etc. The collagen will usually be available in a dispersion ranging from about 5 to 75 mg/ml of insoluble collagen fibrils or from about 1-10 mg/ml of ACS. Desirably, the collagen is present in at least 25 weight % preferably at least 50 weight % as fibrils. The collagen composition will normally be combined with the fibrinogen solution followed by agitation to provide for a uniform dispersion. The composition is then polymerized, by adding thrombin to polymerize the fibrinogen, and by heating with an increase in pH to polymerize the collagen. The polymerization may be performed in a molding apparatus to produce the membrane, if desired. Normally, the collagen will be added prior to the addition of the thrombin which induces polymerization with fibrin formation. Optionally, the thrombin may be added to the combined fibrinogen, collagen and/or buffered salt solution in the molding apparatus.

The collagen may be gelled in many ways, e.g., by exposure to a mildly elevated temperature in the range of about 25 to 40°C, particularly 37°C, and raising the pH from about 1.5-3 to 5 or greater, using an appropriately buffered solution or addition of base. The collagen as employed may be cross-linked, using formalin or other conventional agent, e.g., glutaraldehyde, as a cross-linking agent, where the formalin is combined with the collagen by, for example, immersion in a buffered formaldehyde solution from 0.25 to 10 weight %, typically 0.5 to 4 weight %. Alternatively, the collagen may be cross-linked by exposure to formaldehyde, with or without the presence of an acidic or basic vapor. Cross-linking may be prior to or post gellation.

The media in which the gelation is carried out may include a buffered salt solution, such as Ringer's solution (0.15M NaC1, 0.02M CaC1$_2$ and 0.02M KC1, pH ~ 8), phosphate buffered saline, pH 6.5-8, typically 7-7.5, Tris-Cl, etc. The ionic concentration will be in the range of about 0.15 to 0.4, typically 0.18 to 0.24.

Dehydration may be achieved in a variety of ways, normally under non-denaturing conditions. The gel may be allowed to collapse under the force of gravity or by application of a weight over the gel in an apparatus that allows the water to escape, e.g., a filter plate. Alternatively, evaporation may be employed, using continuous air flow, mildly elevated temperatures, a vacuum, or the like. Other techniques include placing the gelled material over an absorbent gauze, maintaining the gel in the presence of a desiccant, or the like.

The membrane will generally be of a density in the range of from 0. 5 - 50, usually about 1-5 mg protein/cm$^2$. The tear resistance will be at least 10 grams.

Other components may be included in the membrane, depending upon its application. Various therapeutic agents may be employed, such as biocides, particularly antibiotics, bactericides, and growth factors, where the agents may modulate, e.g., enhance or retard, tissue regeneration and growth. These components will generally be present in conventional amounts to provide therapeutic effect.

Other structural molecules and materials may be added to alter the mechanical properties of the basic material. These may include calcium phosphate mineral containing compositions, such as bone powder, hydroxylapatite, tricalcium phosphate, etc., naturally occurring structural polymers, such as elastin, laminin, actin, etc., synthetically produced amino acid polymers, particularly associated with structural polymers, such as silk, keratin, spider silk, etc., naturally occurring polymeric materials other than amino acids, particularly polysaccharides, such as hyaluronic acid, etc. These additives shall not exceed 50 weight %, usually not exceed 20 weight %, more usually not exceed 5 weight %. Other materials may be added to enchance drug release properties such as liposomes, gelatin, polysaccharides, etc.

The subject compositions find a variety of uses, such as hemostatic wound dressings, hemostatic temporary skin grafts, pressure bandages, dressings, and anastomotic splints, particularly when dried in a stretched configuration and allowed to shrink to the original configuration when wet.

Alternately, the gel may be used as formed or shaped into a desireable configuration. These might find use as wound dressings, tissue modifiers, tissue fillers, hemostatic agents, tissue growth modulators, tissue separators or drug delivery implants.

The subject membranes are characterized by being able to be formed in a stretched configuration after molding by tension. Upon wetting, shrinking of from about 5 to 50% of the original volume is obtained. The membrane may also serve as a depository for drugs. The proteinaceous nature of the membrane allows it eventually to be absorbed, to aid in vascularization and angiogenesis at the wound site, and to act as a scaffolding for tissue remodeling or act as a barrier to prevent tissue growth, as in adhesions secondary to surgical procedures.

The following examples are offered by illustration and not by way of limitation.

## EXPERIMENTAL

Production Methods:

The methods listed below are used to produce membranes of about 3 mg protein/cm$^2$ density. This is a useful density to produce compliance in collagen membranes.

Fibrin source is lyophilized bovine fibrinogen from Sigma Chemical Co. (#F4753) dissolved in non-lactated Ringer's solution at a protein concentration of 3 mg/ml. Alternatively, citrated plasma from a variety of sources, e.g., human, bovine, porcine, etc. may be used in the place of the fibrinogen solution.

1. Fibrin Alone

    a. 150 mg fibrinogen + 50 ml Ringer's @ 37°C or 50 ml of citrated plasma.
    b. mix on stir plate until fibrinogen is dissolved.

c. cool solution to 4°C (optional).

d. pour into mold apparatus.

e. add dropwise 8-10 NIH units thrombin (100 unit/ml water solution).

f. mix by gentle swirling.

g. warm to room temperature or 37°C for at least 2 hours, leave in laminar flow hood overnight;

     or

compress gel by using a weight;

     or

leave as is.

2. <u>Fibrin + insoluble collagen fibrils,, 1:1 w/w</u>

a. 75 mg fibrinogen + 25 ml Ringer's @ 37°C.

b. mix on stir plate until fibrinogen is dissolved.

c. cool to 4°C (optional).

d. add 75 mg of insoluble collagen fibrils (2.2 ml of a 35 mg/ml collagen fibril solution).

e. mix on stir plate, pour into mold apparatus.

f. add 8-10 NIH units thrombin, dropwise.

g. warm to room temperature or 37°C, leave in laminar flow hood overnight;

     or

compress gel by using a weight;

     or

leave as is.

3. <u>Fibrin + Acidic Collagen Solution (ACS)</u>

a. 75 mg fibrinogen + 25 ml Ringer's @ 37°C.

b. mix on stir plate until fibrinogen is dissolved.

c. cool to 4°C (optional).

d. (75 mg) 25 ml ASC @ 4°C + 2.5 ml 10X PBS mixed on stir plate.

e. mix fibrinogen and collagen/buffered salt solutions on stir plate.

f. pour mixture into mold apparatus.

g. add 8-10 NIH units thrombin dropwise.

h. mix by gentle stirring.

i. warm to room temperature, incubate 2 hours @ 37°C, leave in laminar flow hood overnight;

     or

compress by using a weight.

4. Plasma Membrane

a. pour 50 ml citrated plasma @4°C into mold apparatus.

b. add 8 - 10 NIH units thrombin dropwise.

c. mix by gentle swirling.

d. warm to room temperature or 37°C for at least two hours.

e. leave in laminar flow hood overnight, or compress gel by weight, dessication, etc;

     or

leave as is.

<u>Properties of the Product:</u>

The properties of the products are described in the following table:

## Table 1

### Tear Resistance[a] of Fibrin/Collagen Membrane[b]

| Material Description | Tear Resistance (grams)[a] |
|---|---|
| 1. fibrin alone | $17.6 \pm 4.7$ |
| 2. 1:1 fibrin + ASC[e] (x-link)[d] | $13.4 \pm 0.6$ |
| 3. 1:1 fibrin + insoluble collagen fibrils | $34.3 \pm 6.0$ |

a. Tear resistance is defined as the weight in grams needed to pull a loop of 4-0 nylon suture through a membrane sample immersed in Phosphate Buffered Saline (PBS) (room temp., pH 7.4). The suture is threaded 5 mm from the edge of the membrane sample. (Values are $\pm$ 1SD)

b. material protein density: 3 mg/cm$^2$

c. all composition ratios are w/w

d. cross-link agent = 4% neutral buffered formalin.

e. ACS = (3mg/ml bovine collagen in 0.012 M HCl) (pH=2).

In Vitro Cell Compatibility Study

Cell culture techniques were utilized to evaluate the effect of bovine collagen, bovine fibrinogen, citrated human plasma, fibrinogen/collagen and plasma/collagen upon cell growth. The bottom of the cell culture well (polystyrene) was used as a control.

The protein concentration of all the solutions was 3 mg/ml. The fibrinogen/collagen solutions were mixed 1:1 w/w. The solutions were mixed by syringe to syringe admixing at 4°C. One part 10X PBS was added to 9 parts acid solubilized collagen. The fibrinogen solution was prepared as previously described. A total of 3 ml of test material was poured into each test well of a 6 well culture plate. Thrombin was added to the fibrinogen containing solutions at a concentration of 0.1 NIH units/ml solution. After pouring the mixed solutions into the test wells and mixing any added thrombin by gentle swirling, the plates were incubated at 37°C for 2 hours.

Three ml of minimal essential media with fetal bovine serum and antibiotic/antimycotic were added to each well. $10^6$ trypsinized rabbit fibroblasts (2nd pass) were then added to each well. The media was changed after 24 hours, then every 48 hours afterwards. The plates were incubated in 5% $CO_2$ at 37° for six days. Sterile technique was utilized for all procedures. The wells were examined by light microscopy at 60X to evaluate confluency, cell morphology and degree of test material surface coverage by the cells.

Control: the fibroblasts were found to be spindle shaped, confluent and completely covering the test surface. No dead cells were observed.

Fibrin: the cell growth was found to be identical to that of the controls; confluent, spindle shaped cells covering the entire surface.

Plasma: mostly spindle shaped cells with confluent growth were found; <<25% cells were individual with dendritic growth. Incomplete coverage of the surface was noted. A few dead cells were found.

Collagen: sparse growth was observed with almost no confluency. Individualized cells with dendritic growth were found comprising >75% of the cell population. <50% of the material surface was covered by cells. A few dead cells were observed.

Fibrin/Collagen: the cell growth was sparse, less than that for the collagen. The cells were rounded in shape and in clumps with no spreading on the material surface. <50% of the material surface area was covered by cells. Numerous dead cells were observed.

Plasma/Collagen: nearly complete confluency was observed. The cells were found to have a spindle morphology comprising >75% of the population. <25% of the cells were found to be individualized with a dendritic morphology. >75% of the material surface area was covered with cells. This material appeared to have better coverage than that of either plasma or collagen individually.

In summary of these findings, the ranking of these materials as compatible for normal cell growth and proliferation from most compatible to least compatible are:

control = fibrin > plasma/collagen > plasma > collagen > fibrin/collagen.

The above results show that useful gel membranes, having good tear resistance, may be obtained by gelling fibrinogen with thrombin by itself or in combination with collagen. They can be prepared in a stretched conformation so as to shrink when wet, and may be formulated with a variety of pharmacologically active compounds, to provide for protection against infection. Structural additives may be incorporated to alter the mechanical properties of the basic material as well as to modify drug release capabilities. The material may also be utilized as a gel. Fibrin allows for good cell growth while fibrin/collagen combinations may be used to inhibit cellular growth.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

1. A method of preparing a proteinaceous membrane free of viable cells having a density in the range of about 0.5 to 50 mg/cm$^2$ which method comprises:

   forming an aqueous composition comprising fibrinogen with 0 to 3 parts by weight of collagen per part of fibrinogen and an aqueous buffered salt-containing medium which is at an acidic pH when acid-soluble collagen is present; and

   polymerising the composition to form said proteinaceous membrane.

2. A method as claimed in claim 1, wherein the fibrinogen present in the composition is polymerised with thrombin at temperatures from 0 or 2°C up to 37 or 40°C, preferably at about 5°C (i.e. 3 to 7°C).

3. A method as claimed in claim 1 or claim 2, wherein the weight ratio of fibrinogen to collagen is 1:0.5 to 1.

4. A method as claimed in any one of the preceding claims wherein said collagen is cross-linked acidic collagen and the collagen is polymerised with a pH of the medium of at least 5 at a temperature of from 25 to 40°C.

5. A method as claimed in any one of the preceding claims wherein after polymerisation of fibrinogen the membrane is formed by dehydrating in a mold under non-denaturing conditions.

6. A method as claimed in any one of the preceding claims wherein the membrane produced comprises fibrinogen as the only protein.

7. A membrane when produced by any one of the preceding claims which comprises an effective amount of at least one therapeutic agent or drug.

8. A membrane as claimed in claim 7, which comprises as a therapeutic agent an agent for inhibiting pathogenic infection.

9. A membrane as claimed in claim 7 comprising at least one agent for modulating tissue growth.

10. A membrane as claimed in any one of claims 7,8 or 9, which comprises in an amount to enhance mechanical properties at least one of a structural protein, a calcium phosphate mineral, hyaluronic acid, bone powder or a synthetic amino acid polymer.